(19) Europäisches Patentamt
European Patent Office
Office européen des brevets

(11) Veröffentlichungsnummer: **0 105 160**
**A1**

(12) # EUROPÄISCHE PATENTANMELDUNG

(21) Anmeldenummer: 83108190.6

(22) Anmeldetag: 19.08.83

(51) Int. Cl.³: C 11 D 3/08, C 11 D 3/12, B 01 F 17/54, A 61 K 7/16

(30) Priorität: 30.09.82 DE 3236180

(43) Veröffentlichungstag der Anmeldung: 11.04.84 Patentblatt 84/15

(84) Benannte Vertragsstaaten: AT BE CH FR GB IT LI NL SE

(71) Anmelder: Akzo GmbH,
Postfach 10 01 49 Kasinostrasse 19-23,
D-5600 Wuppertal-1 (DE)

(72) Erfinder: Adam, Wolfgang, Dr.Dipl.-Chem., Schelle 37,
D-5160 Düren-Derichsweiler (DE)

(54) **Mit Tensid beladene Kieselsäure oder Silicate, Verfahren zu deren Herstellung und deren Verwendung.**

(57) Mit Tensid beladene, frei fliessende Kieselsäure bzw. Silicate, die mit dem 0,7- bis 2,0fachen des gemessenen Wasserporenvolumens einer wässrigen Lösung eines anionenaktiven Tensides beladen ist, wobei das Wasserporenvolumen durch die wässrige Phase der Lösung angefüllt und die Tensid-Phase des anionischen Tensides darüber hinaus aufgenommen ist. Die Kieselsäure bzw. das Silicat weist vorzugsweise eine spezifische Oberfläche von 50–750 m²/g, ein spezifisches Wasserporenvolumen von 0,1 bis 2,5 cm³/g und eine mittlere Partikelgrösse von 5 bis 50 µm auf, wobei der Gehalt an organischen Nebenprodukten aus der Tensidherstellung weniger als 3,0 Gew.-%, bezogen auf die Aktivsubstanz des Tensides beträgt. Sie wird dadurch hergestellt, dass auf eine getrocknete Kieselsäure bzw. Silicat mit einem Wassergehalt von weniger als 20 Gew.-% einer wässrigen 5 bis 50 Gew.-% Aktivsubstanz enthaltenden anionenaktiven Tensidlösung aufgesprüht wird. Sie wird als oder in Reinigungs-, Schleif- und Poliermittel, insbesondere als oder in Zahnpflegemittel verwendet.

EP 0 105 160 A1

# Mit Tensid beladene Kieselsäure oder Silicate, Verfahren zu deren Herstellung und deren Verwendung.

Akzo GmbH

Wuppertal

* * *

Die Erfindung betrifft mit Tensid beladene Kieselsäure oder Silicate, ein Verfahren zu deren Herstellung und deren Verwendung.

Es ist bekannt, Kieselsäure als Abrasivum für Reinigungs-, Polier- und Schleifmittel einzusetzen. Ebenfalls ist es bekannt, auch für Zahnpasten Kieselsäure als Abrasivum einzusetzen. Für solche Einsatzzwecke wird zusammen mit dem Abrasivum ein Tensid verwendet, um den notwendigen Schaum zu erzeugen, der die abrasiven Produkte gleichmäßig verteilt.

Es gibt einen umfangreichen Stand der Technik, der sich mit Kieselsäure als Abrasivum befaßt, wobei die abrasiven Eigenschaften im Vordergrund stehen. So empfiehlt die DE-B-16 17 927 Kieselsäurexerogele mit einer Teilchengröße von 2 bis 20 µm und einer spezifischen Oberfläche von mindestens 600 m²/g. Nach der DE-A- 20 28 866 soll jedoch die Oberfläche nicht kritisch sein und auch 300 bis 500 m²/g betragen können.

Nach der DE-A- 16 67 875 sollen pyrogene hydrophobe Kieselsäuren brauchbar sein, deren Primärteilchengröße nur etwa 0,01 bis 0,03 µm beträgt. Andererseits heißt es in der DE-A- 22 50 078, daß die Teilchengröße auch 20 µm überschreiten kann; es werden dort Kieselsäurexerogele mit spezifischen Oberflächen zwischen 250 und 800 m²/g empfohlen, deren Teilchengröße zwischen 22 und 50, insbesondere 25 und 30 µm liegt.

In der DE-B- 24 46 038 wird das größte Gewicht auf die Schüttdichte gelegt und gezeigt, daß das Abriebvermögen (bestimmt als Drahtabrieb) mit ansteigendem Schüttgewicht ebenfalls zunimmt. Dabei wird allerdings nicht deutlich, wie die verschiedenen Schüttgewichte erhalten werden können. In den Beispielen zeigt die spezifische Oberfläche keine Korrelation mit der Scheuerwirkung oder mit dem Schüttgewicht.

Die DE-A- 27 04 504 empfiehlt Kieselsäuren mit einem Wassergehalt von 15 bis 35 Gew.-% mit einer Teilchengröße im Bereich von 2 bis 30 µm.

Die DE-A- 25 22 486 strebt Kieselsäuren mit einer niedrigen Struktur an, worunter eine geringe Ölaufnahme bei hoher Schüttdichte verstanden werden soll.

Aus der DE-B- 11 15 725 ist es bekannt, dem nassen Filterkuchen Tenside zuzusetzen, die nur im alkalischen Bereich beständig sind. Dieser Zusatz dient der Verbesserung des Auswaschens von Elektrolyten nach dem Fällen der Kieselsäure. Beim anschließenden Ansäuern der Dispersion werden die Tenside gespalten, so daß die Kieselsäure nach dem Trocknen in den Poren keine anionenaktiven Tenside enthält.

Die DE-A- 28 53 647 beschreibt die Herstellung einer Zahnpaste, wobei ein Gemisch aus einem Kieselgel, einem handelsüblichen Kieselsäurefüllstoff mit einem Wassergehalt von 50 % und einem handelsüblichen Aerogel mit den übrigen Bestandteilen zu der Zahnpaste gemischt werden. Als Mischungsbestandteil ist auch Na-Laurylsulfat erwähnt. Eine mit Tensid beladene Kieselsäure wird dabei jedoch nicht erhalten.

Aufgabe der vorliegenden Erfindung war es, eine mit Tensiden beladene Kieselsäure oder Silicate zu schaffen, die als Pulver fließfähig sind und die in einfacher Weise durch Zumischen der übrigen üblichen Mischungsbestandteile zu Reinigungs-, Schleif- und Poliermitteln insbesondere auch zu Zahnpflegemittel verwendet werden kann. Unter Kieselsäuren sollen insbesondere die gefällten Kieselsäuren und mehr oder weniger hydratisiertes $SiO_2$ verstanden werden. Dabei sollen auch Silicate mit umfaßt werden, die sich in den adsorptiven Eigenschaften analog wie Kieselsäure verhalten.

Diese Aufgabe wird dadurch gelöst, daß Kieselsäure oder ein Silicat mit dem 0,7- bis 2,0fachen des gemessenen Wasserporenvolumens einer wäßrigen Lösung eines anionenaktiven Tensides beladen ist, wobei das Wasserporenvolumen durch die wäßrige Phase der Lösung angefüllt und die Tensid-Phase des anionischen Tensides darüber hinaus aufgenommen ist.

Das Wasserporenvolumen wird in der nachfolgend beschriebenen Weise bestimmt.

5 g Kieselsäure werden in einen Weithalskolben gegeben. Aus einer Bürette wird langsam Wasser zugetropft und durch leichtes Schütteln und Aufschlagen des Kolbens auf einen Gummistopfen verteilt.

Wenn das Produkt beginnt, an den Wandungen zu kleben, ist es mit Wasser gesättigt. Das aufgenommene Volumen Wasser, bezogen auf 1 g Substanz, ist das Wasserporenvolumen.

Vorzugsweise ist die mit Tensid beladene, frei fließende Kieselsäure bzw. Silicate dadurch gekennzeichnet, daß die Poren einer Kieselsäure oder eines Silicates, eine spezifische Oberfläche von 50 - 750 $m^2/g$, ein spezifisches Wasserporenvolumen von 0,1 bis 2,5 $cm^3/g$ und eine mittlere Partikelgröße von 5 bis 50 µm aufweisen, wobei der Gehalt an organischen Nebenprodukten aus der Tensidherstellung weniger als 3,0 Gew.-%, bezogen auf die Aktivsubstanz des Tensides beträgt.

Handelsübliche anionenaktive Tenside mit einem Gehalt an Aktivsubstanz von ca. 30 % haben herstellungsbedingt einen Gehalt von mehr als 1,0 - 1,5 Gew.-% organischen Nebenprodukten, beispielsweise freiem Fettalkohol und/oder freiem Fettalkoholäther und/oder freien Kohlenwasserstoffen. Das entspricht beispielsweise einem Gehalt an freiem Alkohol von 3,3 - 5,0 % auf die Aktivsubstanz bezogen. Wegen der Geschmacksbeeinträchtigungen bei Zahnpflegemitteln - Laurylalkohol schmeckt stark bitter - werden heute in Zahnpflegemitteln durchweg sprühgetrocknete Laurylalkoholsulfate eingesetzt, bei denen der Gehalt an freiem Laurylalkohol durch den Sprühprozeß verringert worden ist. Die erfindungsgemäße mit Tensiden beladene Kieselsäure bzw. Silicate soll daher einen möglichst geringen Gehalt an solchen herstellungsbedingten Nebenprodukten aufweisen, so daß vorzugsweise der Gehalt an ihnen weniger als 3 Gew.-%, vorzugsweise weniger als 2 Gew.-%, bezogen auf die Aktivsubstanz des Tensides beträgt.

Solche geringen Gehalte an organischen Nebenprodukten in einer anionenaktiven Tensid-Lösung können mit speziellen Sulfierverfahren auch ohne Sprühtrocknung erreicht werden.

In einer Ausführungsform der Erfindung ist das anionenaktive Tensid ein Fettalkoholsulfat eines Fettalkohols mit 10 - 18 C-Atomen, vorzugsweise Laurylalkoholsulfat.

In einer anderen Ausführungsform ist das anionenaktive Tensid ein Fettalkoholäthersulfat eines äthoxilierten Fettalkohols mit 10 - 18 C-Atomen in der Kohlenstoffkette und 1 - 6 Mol Äthylenoxid, vorzugsweise Lauryläthersulfat.

In einer weiteren Ausführungsform der Erfindung ist das anionenaktive Tensid ein Olefinsulfonat mit 12 bis 18 C-Atomen, wobei vorzugsweise die durchschnittliche Kettenlänge des Olefinsulfonates 14 bis 16 C-Atome beträgt.

In einer anderen Ausführungsform der Erfindung ist das anionenaktive Tensid ein Paraffinsulfonat mit 11 bis 18 C-Atomen.

Die Tenside können als wäßrige Lösung eines der vorgenannten anionenaktiven Tenside eingesetzt werden. Anpassungen an besondere Einsatzgebiete ergeben sich daraus, daß die wäßrige Tensidlösung ein Gemisch aus mehreren anionaktiven Tenside enthält.

Im allgemeinen ist die Kieselsäure bzw. das Silicat mit einer Tensidlösung beladen die 5 bis 50 Gew.-% des anionenaktiven Tensides enthält.

Bei manchen Tensiden ergeben sich Schwierigkeiten bei der Beladung der Kieselsäure aufgrund der hohen Viskosität mancher Tensidlösungen, die einen hohen Aktivgehalt aufweisen. Andererseits ist es erwünscht, daß die Kieselsäure bzw. das Silicat eine möglichst hohe Beladung mit Tensiden erhält. Deshalb ist es im Rahmen der Erfindung bevorzugt, daß die Kieselsäure mit einer Tensidlösung beladen ist, die 20 bis 40 Gew.-% des anionenaktiven Tensides enthält.

Hergestellt werden die erfindungsgemäßen Produkte mittels eines Verfahrens, das dadurch gekennzeichnet ist, daß auf eine Kieselsäure oder ein Silicat das 0,7- bis 2,0fache des gemessenen Wasserporenvolumens einer wäßrigen, ein anionenaktives Tensid enthaltenden Tensidlösung aufgesprüht wird.

Vorzugsweise wird auf eine getrocknete Kieselsäure bzw. ein Silicat mit einem Wassergehalt von weniger als 20 Gew.-%, mit einer spezifischen Oberfläche von 50 - 750 $m^2/g$ und einem spezifischen Wasserporenvolumen von 0,1 - 2,5 $cm^3/g$ einer wäßrigen 5 bis 50 Gew.-% Aktivsubstanz enthaltenden anionenaktiven Tensidlösung aufgesprüht.

Als Kieselsäuren können dabei Hydrogele und Xerogele mit den entsprechenden spezifischen Oberflächen und spezifischen Wasservolumina eingesetzt werden.

Vorzugsweise wird erfindungsgemäß eine wäßrige 20 bis 40 Gew.-% Aktivsubstanz enthaltende, wäßrige Tensidlösung in einer Menge des 1,0- bis 1,7fachen Wasserporenvolumens der Kieselsäure bzw. des Silicates aufgesprüht.

Eine bevorzugte Ausführungsform des erfindungsgemäßen Verfahrens besteht darin, daß eine wäßrige 25 - 35 Gew.-% Aktivsubstanz enthaltende Lösung eines anionenaktiven Tensides in einer Menge des 1,2- bis 1,55fachen Wasserporenvolumens der Kieselsäure bzw. des Silicates aufgesprüht wird. Vorzugsweise ist das anionenaktive Tensid ein Fettalkoholsulfat mit 10 - 18 C-Atomen, insbesondere ein Laurylalkoholsulfat.

Andere bevorzugte erfindungsgemäße Ausführungsformen ergeben sich dadurch, daß als anionenaktive Tenside Fettalkoholäthersulfate von äthoxilierten Fettalkoholen mit 10 - 18 C-Atomen in der Kohlenstoffkette und 1 - 6 Mol Äthylenoxid insbesondere ein Lauryläthersulfat, Olefinsulfonate mit 12 - 18 C-Atomen, vorzugsweise 14 bis 16 C-Atomen, und/oder Paraffinsulfonate mit 11 - 18 C-Atomen eingesetzt werden.

Die Tenside können einzeln oder als Gemisch verschiedener anionenaktiver Tenside eingesetzt werden.

Der Gehalt an herstellungsbedingten organischen Nebenprodukten soll bei den Tensiden weniger als 3 Gew.-%, vorzugsweise weniger als 2 Gew.-%, bezogen auf die Aktivsubstanz betragen.

Obwohl bei dem erfindungsgemäßen Verfahren erwartet werden mußte, daß ein relativ klebriges Produkt entsteht, zeigte es sich, daß aus der vorher sehr staubigen, trockenen Kieselsäure ein freifließendes mehliges Produkt entstand, welches je nach verwendeter Tensid-Lösung 6 - 20 Gew.-% anionische Substanz enthielt, deren Gehalt sich ohne Schwierigkeit durch Titration feststellen ließ. Dabei zeigen weder die verschiedenen verwendeten Kieselsäuren, noch Silicate, noch Natriumaluminiumsilicat prinzipielle Unterschiede.

Das erfindungsgemäße Produkt bringt nicht nur technische Vorteile bei der Weiterverarbeitung, wie geringeres Stauben, sondern auch wirtschaftliche Vorteile mit sich, die darin liegen, daß die Verpackungs-, Transport- und Lagerkosten deutlich gesenkt werden.

Anhand der nachfolgenden Beispiele wird die Erfindung näher erläutert.

Beispiel:

Es wurden Kieselsäuren bzw. Aluminiumsilicate mit den in der nachfolgenden Tabelle zusammengestellten Kenndaten eingesetzt

Tabelle 1

| Bez. | Chemische Zusammensetzung | spez. Oberfläche $m^2/g$ | Wassergehalt Gew.-% | mittlere Teilchengröße (µm) | Wasserporenvolumen ml/g |
|---|---|---|---|---|---|
| A | $SiO_2$ | 130 | 6 | 20 | 1,39 |
| B | $SiO_2$ | 150 | 6 | 20 | 1,22 |
| C | $SiO_2$ | 150 | 6 | 8 | 1,40 |
| D | $SiO_2$ | 130 | 6 | 8 | 1,40 |
| E | NaAl-Silicat | 110 | 7 | 25 | 1,0 |
| F | $SiO_2$ | 500 | 4 | 20 | 0,7 |

Je 200 g der zuvor gekennzeichneten Kieselsäuren bzw. Silicate wurden mit den jeweils angeführten Mengen einer 30prozentigen wäßrigen Lösung einer Tensidlösung beladen. Als Tensid diente bei diesen Beispielen Natriumlaurylsulfat. Die Kieselsäuren wurden in ein Becherglas gegeben, das langsam schräg rotierte und die Tensidlösung wurde langsam aufgetropft. Sie kann auch zur besseren Verteilung aufgesprüht werden. Nach Beendigung der Tensidlösungszugabe wurde das noch etwas inhomogene Produk kurz in einen Mischer mit rotierendem Messerkopf (Schlagkrenzmühle) homogenisiert. Es entstand ein freifließendes, mehliges, staubfreies Pulver soweit nicht anders angegeben ist, welches unmittelbar als und in üblichen Rezepturen zu Reinigungs-, Schleif- und Poliermitteln, insbesondere zu Zahnpflegemitteln hervorragend geeignet war. In einer Zweiphasentitration, wie sie für anionenaktive Tenside üblich ist, konnte der Gehalt an waschaktiver Substanz in guter Übereinstimmung mit der Theorie ermittelt werden. Die erhaltenen Ergebnisse sind in der nachfolgenden Tabelle 2 zusammengestellt.

Tabelle 2

| Versuch Nr. | verwendete Kieselsäure | Menge Tensidlösung ml | bezogen auf WPV % | WAS % | Beurteilung des Produktes | Eignung |
|---|---|---|---|---|---|---|
| 1 | A | 265 | 95 | 17,1 | trockenes Pulver frei fließend | ++ |
| 2 | B | 220 | 90 | 15,7 | trockenes Pulver frei fließend | ++ |
| 3 | B | 340 | 139 | 18,9 | trockenes Pulver frei fließend | + |
| 4 | E | 400 | 200 | 20,0 | trockenes Pulver frei fließend | + |
| 5 | C | 230 | 82 | 16,1 | trockenes Pulver frei fließend | ++ |
| 6 | C | 435 | 155 | 20,1 | trockenes Pulver frei fließend | + |
| 7 | C | 470 | 167 | 21,0 | klebriges Produkt/ nicht frei fließend | - |
| 8 | F | 165 | 118 | 13,6 | gut mahlbares, frei fließendes Pulver | ++ |
| 9 | D | 400 | 143 | 20,0 | gut mahlbares, frei fließendes Pulver | ++ |

++ sehr gute Eignung, + gute Eigung, - ungeeignet.

WPV = Wasserporenvolumen

## Patentansprüche

1. Mit Tensid beladene, frei fließende Kieselsäure oder frei fließende Silicate, dadurch gekennzeichnet, daß sie mit dem 0,7- bis 2,0fachen des gemessenen Wasserporenvolumens einer wäßrigen Lösung eines anionenaktiven Tensides beladen sind, wobei das Wasserporenvolumen durch die wäßrige Phase der Lösung angefüllt und die Tensid-Phase des anionischen Tensides darüber hinaus aufgenommen ist.

2. Mit Tensid beladene Kieselsäure oder Silicate nach Anspruch 1, dadurch gekennzeichnet, daß die Poren einer Kieselsäure oder eines Silicates eine spezifische Oberfläche von 50 bis 750 $m^2/g$, ein spezifisches Wasserporenvolumen von 0,1 bis 2,5 $cm^3/g$, und eine mittlere Partikelgröße von 5 bis 50 µm aufweisen, wobei der Gehalt an organischen Nebenprodukten aus der Tenidherstellung weniger als 3,0 Gew.-%, bezogen auf die Aktivsubstanz des Tensides beträgt.

3. Mit Tensid beladene Kieselsäure oder Silicate nach den Ansprüchen 1 bis 2, dadurch gekennzeichnet, daß das anionenaktive Tensid ein Fettalkoholsulfat eines Fettalkohols mit 10 bis 18 C-Atomen ist.

4. Mit Tensid beladene Kieselsäure oder Silicate nach Anspruch 3, dadurch gekennzeichnet, daß das anionenaktive Tensid ein Laurylalkoholsulfat ist.

5. Mit Tensid beladene Kieselsäure oder Silicate nach den Ansprüchen 1 bis 2, dadurch gekennzeichnet, daß das anionenaktive Tensid ein Fettalkoholäthersulfat eines äthoxilierten Fettalkohols mit 10 bis 18 C-Atomen in der Kohlenstoffkette und 1 bis 6 Mol Äthylenoxid ist.

6. Mit Tensid beladene Kieselsäure oder Silicate nach Anspruch 5, dadurch gekennzeichnet, daß das Fettalkoholäthersulfat Lauryläthersulfat ist.

7. Mit Tensid beladene Kieselsäure oder Silicate nach den Ansprüchen 1 bis 2, dadurch gekennzeichnet, daß das anionenaktive Tensid ein Olefinsulfonat mit 12 bis 18 C-Atomen ist.

8. Mit Tensid beladene Kieselsäure oder Silicate nach Anspruch 7, dadurch gekennzeichnet, daß die durchschnittliche Kettenlänge des Olefinsulfonates 14 bis 16 C-Atome beträgt.

9. Mit Tensid beladene Kieselsäure oder Silicate nach den Ansprüchen 1 bis 2, dadurch gekennzeichnet, daß das anionenaktive Tensid ein Paraffinsulfonat mit 11 bis 18 C-Atomen ist.

10. Mit Tensid beladene Kieselsäure nach den Ansprüchen 1 bis 9, dadurch gekennzeichnet, daß die wäßrige Tensidlösung ein Gemisch aus mehreren anionenaktiven Tensiden enthält.

11. Mit Tensid beladene Kieselsäure oder Silicate nach den Ansprüchen 1 bis 10, dadurch gekennzeichnet, daß der Gehalt an organischen Nebenprodukten aus der Tensidherstellung weniger als 2 Gew.-%, bezogen auf die Aktivsubstanz des Tensides beträgt.

12. Mit Tensid beladene Kieselsäure nach den Ansprüchen 1 bis 11, dadurch gekennzeichnet, daß die Tensidlösung 5 bis 50 Gew.-% des anionenaktiven Tensides enthält.

13. Mit Tensid beladene Kieselsäure oder Silicate nach Anspruch 12, dadurch gekennzeichnet, daß die Tensidlösung 20 bis 40 Gew.-% des anionenaktiven Tensides enthält.

14. Verfahren zur Herstellung der mit Tensid beladenen Kieselsäure oder Silicate nach den Ansprüchen 1 bis 13, dadurch gekennzeichnet, daß auf eine Kieselsäure oder ein Silicat das 0,7- bis 2,0fache des gemessenen Wasserporenvolumens einer wäßrigen, ein anionenaktives Tensid enthaltenden Tensidlösung aufgesprüht wird.

15. Verfahren nach Anspruch 14, dadurch gekennzeichnet, daß auf eine getrocknete Kieselsäure oder ein getrocknetes Silicat mit einem Wassergehalt von weniger als 20 Gew.-%, mit einer spezifischen Oberfläche von 50 bis 750 $m^2/g$ und einem spezifischen Wasserporenvolumen von 0,1 bis 2,5 $cm^3/g$ einer wäßrigen 5 bis 50 Gew.-% Aktivsubstanz enthaltenden anionenaktiven Tensidlösung aufgesprüht wird.

16. Verfahren nach den Ansprüchen 14 bis 15, dadurch gekennzeichnet, daß eine wäßrige 20 bis 40 Gew.-% Aktivsubstanz enthaltende, wäßrige Tensidlösung in einer Menge des 1,0- bis 1,7fachen Wasserporenvolumens der Kieselsäure oder des Silicates aufgesprüht wird.

17. Verfahren nach Anspruch 16, dadurch gekennzeichnet, daß eine 25 bis 35 Gew.-% Aktivsubstanz enthaltende, wäßrige Tensidlösung in einer Menge des 1,2- bis 1,55fachen Wasserporenvolumens der Kieselsäure oder des Silicates aufgesprüht wird.

18. Verfahren nach den Ansprüchen 14 bis 17, dadurch gekennzeichnet, daß das anionenaktive Tensid ein Fettalkoholsulfat eines Fettalkohols mit 10 bis 18 C-Atomen ist.

19. Verfahren nach Anspruch 18, dadurch gekennzeichnet, daß das anionenaktive Tensid ein Laurylalkoholsulfat ist.

20. Verfahren nach den Ansprüchen 14 bis 17, dadurch gekennzeichnet, daß das anionanaktive Tensid ein Fettalkoholäthersulfat eines äthoxilierten Fettalkohols mit 10 bis 18 C-Atomen in der Kohlenstoffkette und 1 bis 6 Mol Äthylenoxid ist.

21. Verfahren nach Anspruch 20, dadurch gekennzeichnet, daß das Fettalkoholäthersulfat Lauryläthersulfat ist.

22. Verfahren nach den Ansprüchen 14 bis 17, dadurch gekennzeichnet, daß das anionenaktive Tensid ein Olefinsulfonat mit 12 bis 18 C-Atomen ist.

23. Verfahren nach Anspruch 22, dadurch gekennzeichnet, daß die durchschnittliche Kettenlänge des Olefinsulfontes 14 bis 16 C-Atome beträgt.

24. Verfahren nach den Ansprüchen 14 bis 17, dadurch gekennzeichnet, daß das anionenaktive Tensid ein Paraffinsulfonat mit 11 bis 18 C-Atomen ist.

25. Verfahren nach den Ansprüchen 14 bis 24, dadurch gekennzeichnet, daß die wäßrige Tensidlösung ein Gemisch aus mehreren anionenaktiven Tensiden enthält.

26. Verfahren nach den Ansprüchen 14 bis 25, dadurch gekennzeichnet, daß ein Tensid eingesetzt wird dessen Gehalt an organischen Nebenprodukten aus der Tensidherstellung weniger als 3 Gew.-%, bezogen auf die Aktivsubstanz der Tenside beträgt.

27. Verfahren nach Anspruch 26, dadurch gekennzeichnet, daß der Gehalt an organischen Nebenprodukten aus der Tensidherstellung weniger als 2 Gew.-% beträgt.

28. Verwendung der mit Tensid beladenen Kieselsäure oder Silicate nach den Ansprüchen 1 bis 13 oder hergestellt nach den Ansprüchen 14 bis 27 als oder in Reinigungs-, Schleif- und Poliermittel.

29. Verwendung der mit Tenid beladenen Kieselsäure oder Silicate nach Anspruch 28 als oder in Zahnpflegemittel.

## Patentansprüche AT

1. Verfahren zur Herstellung von mit Tensid beladenen Kieselsäuren oder Silicaten, dadurch gekennzeichnet, daß auf eine Kieselsäure oder ein Silicat das 0,7- bis 2,0fache des gemessenen Wasserporenvolumens einer wäßrigen, ein anionenaktives Tensid enthaltenden Tensidlösung aufgesprüht wird.

2. Verfahren nach Anspruch 1, dadurch gekennzeichnet, daß auf eine getrocknete Kieselsäure oder ein getrocknetes Silicat mit einem Wassergehalt von weniger als 20 Gew.-%, mit einer spezifischen Oberfläche von 50 - 750 $m^2/g$ und einem spezifischen Wasserporenvolumen von 0,1 - 2,5 $cm^3/g$ einer wäßrigen 5 bis 50 Gew.-% Aktivsubstanz enthaltenden anionenaktiven Tensidlösung aufgesprüht wird.

3. Verfahren nach den Ansprüchen 1 bis 2, dadurch gekennzeichnet, daß eine wäßrige 20 bis 40 Gew.-% Aktivsubstanz enthaltende, wäßrige Tensidlösung in einer Menge des 1,0- bis 1,7fachen Wasserporenvolumens der Kieselsäure oder des Silicates aufgesprüht wird.

4. Verfahren nach Anspruch 3, dadurch gekennzeichnet, daß eine 25 bis 35 Gew.-% Aktivsubstanz enthaltende, wäßrige Tensidlösung in einer Menge des 1,2- bis 1,55fachen Wasserporenvolumens der Kieselsäure oder des Silicates aufgesprüht wird.

5. Verfahren nach den Ansprüchen 1 bis 4, dadurch gekennzeichnet daß das anionenaktive Tensid ein Fettalkoholsulfat eines Fettalkohols mit 10 bis 18 C-Atomen ist.

6. Verfahren nach Anspruch 5, dadurch gekennzeichnet, daß das anionenaktive Tensid ein Laurylalkoholsulfat ist.

7. Verfahren nach den Ansprüchen 1 bis 4, dadurch gekennzeichnet, daß das anionenaktive Tensid ein Fettalkoholäthersulfat eines äthoxilierten Fettalkohols mit 10 bis 18 C-Atomen in der Kohlenstoffkette und 1 bis 6 Mol Äthylenoxid ist.

8. Verfahren nach Anspruch 7, dadurch gekennzeichnet, daß das Fettalkoholäthersulfat Laurylächersulfat ist.

9. Verfahren nach den Ansprüchen 1 bis 4, dadurch gekennzeichnet, daß das anionenaktive Tensid ein Olefinsulfonat mit 12 bis 18 C-Atomen ist.

10. Verfahren nach Anspruch 9, dadurch gekennzeichnet, daß die durchschnittliche Kettenlänge des Olefinsulfonates 14 bis 16 C-Atome beträgt.

11. Verfahren nach den Ansprüche 1 bis 4, dadurch gekennzeichnet, daß das anionenaktive Tensid ein Paraffinsulfonat mit 11 bis 18 C-Atomen ist.

12. Verfahren nach den Ansprüchen 1 bis 11, dadurch gekennzeichnet, daß die wäßrige Tensidlösung ein Gemisch aus mehreren anionenaktiven Tensiden enthält.

13. Verfahren nach den Ansprüchen 1 bis 12, dadurch gekennzeichnet, daß ein Tensid eingesetzt wird, dessen Gehalt an organischen Nebenprodukten aus der Tensidherstellung weniger als 3 Gew.-%, bezogen auf die Aktivsubstanz der Tenside beträgt.

14. Verfahren nach Anspruch 13, dadurch gekennzeichnet, daß der Gehalt an organischen Nebenprodukten aus der Tensidherstellung weniger als 2 Gew.-% beträgt.

15. Verwendung der mit Tensid beladenen Kieselsäure oder Silicate hergestellt nach den Ansprüchen 1 bis 14 als oder in Reinigungs-, Schleif- und Poliermittel.

16. Verwendung der mit Tensid beladenen Kieselsäure oder Silicate nach Anspruch 15 als oder in Zahnpflegemittel.

0105160

Europäisches Patentamt

# EUROPÄISCHER RECHERCHENBERICHT

Nummer der Anmeldung

EP 83 10 8190

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,A | DE-A-2 853 647 (GRACE GMBH)<br>* Ansprüche 1, 8 * | | C 11 D 3/08<br>C 11 D 3/12<br>B 01 F 17/54<br>A 61 K 7/16 |
| D,A | DE-A-2 704 504 (W.R. GRACE & CO.)<br>* Ansprüche 1, 2 * | | |
| A | US-A-3 934 000 (J.B. BARTH)<br>* Ansprüche 1, 2 * | | |
| A | CH-A- 588 281 (UNILEVER N.V.)<br>* Spalte 2, Beispiel; Anspruch * | | |
| A | US-A-4 108 978 (S. MAZZANOBILE et al.)<br>* Anspruch 1 * | | **RECHERCHIERTE SACHGEBIETE (Int. Cl. 3)**<br>C 11 D 3/00<br>B 01 F 17/00<br>A 61 K 7/00 |

Der vorliegende Recherchenbericht wurde für alle Patentansprüche erstellt.

| Recherchenort | Abschlußdatum der Recherche | Prüfer |
|---|---|---|
| BERLIN | 01-12-1983 | SCHULTZE D |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503 03 82